# EUROPÄISCHE PATENTSCHRIFT

(11) **EP 1 151 293 B1**
(45) Veröffentlichungstag und Bekanntmachung des Hinweises auf die Patenterteilung: **28.04.2004**
(21) Anmeldenummer: 99967002.9
(22) Anmeldetag: 23.12.1999
(51) Int. Cl.: G01N 33/28, G01N 27/00, G01N 29/02

(54) **VERFAHREN ZUR BESTIMMUNG DER ÖLQUALITÄT SOWIE ÖLQUALITÄTSSENSOR**
METHOD FOR DETERMINING OIL QUALITY AND OIL QUALITY SENSOR
PROCEDE POUR DETERMINER LA QUALITE D'HUILE ET DETECTEUR DE QUALITE D'HUILE

(30) Priorität: 19.01.1999 DE 19901815
(43) Veröffentlichungstag der Anmeldung: 07.11.2001
(73) Patentinhaber: Volkswagen Aktiengesellschaft, 38436 Wolfsburg (DE)
(72) Erfinder: DICKERT, Franz, Ludwig, A-3400 Kloserneuburg (AT); GREIBL, Wolfgang, 1170 Wien (AT)
(74) Vertreter: Fritz, Edmund Lothar, Dipl.-Chem.
(86) Internationale Anmeldenummer: PCT/EP1999/010351
(87) Internationale Veröffentlichungsnummer: WO 2000/043773

(56) Entgegenhaltungen:
- WO-A-98/19156
- DE-A- 4 309 660
- US-A- 5 151 110
- US-A- 5 201 215
- US-A- 5 204 381

## Beschreibung

Die Erfindung betrifft einen Ölqualitätssensor gemäß dem Oberbegriff des Anspruchs 8 sowie ein Verfahren zur Bestimmung der Ölqualität nach dem Oberbegriff des Anspruchs 1.

Motorenöle haben sich im Laufe der Zeit zu High-Tech-Produkten entwickelt, die mit Voraussetzung sind, hohe Motorleistungen zu ermöglichen. "Motorenöle" stellen dabei eine Sammelbezeichnung für Grundölkomponenten aus Mineralöl, Hydrocrackanten und synthetischen Komponenten dar. Motorenöle enthalten noch Additive, die als Fertigmischung ("Paket") zugegeben werden, sowie Viskositätsindex-Verbesserer (VI). Die Motorenöle dienen dabei als Schmierstoffe für die Motoren sowie als Kühl- und Abdichtmedium. Weiterhin sollen sie sämtliche Motorenteile reinigen und rein halten. Die VI-Verbesserer bewirken ein günstigeres Viskositäts-Termperaturverhalten, als es die reinen Grundöle aufweisen. Der Anteil an Additiven und VI-Verbesserer liegt je nach Anforderungen, die an das Öl gestellt werden, üblicherweise zwischen 5 und 25 %.

Weitere Aufgaben der Additive in den Motorenölen sind die Verbesserung der Korrosionsschutzeigenschaften der Öle sowie die Verhinderung von Schlammablagerungen und Öleindickungen sowie der Verschleißschutz an den Reibpartnem unter allen auftretenden Belastungen. Die thermischen Beanspruchungen der Motorenöle sind hoch und liegen dabei im Ölsumpf durchschnittlich bei ca. 100 bis 150 °C. Im Bereich der oberen Kolbenringzone können Temperaturspitzen zwischen 200 und 350 °C auftreten.

Im Laufe ihres Gebrauchs altern die Öle, wobei in erster Linie die Additivkomponenten und die VI-Verbesserer abgebaut (verbraucht) werden. Einen erheblichen Anteil an der Öltalterung haben unverbrauchte, teilweise oxidierte und polymerisierte Kraftstoffkomponenten. Somit wird die Ölalterung durch Temperatureinwirkung und reaktionsfähige Verbrennungsprodukte (Radikale) sowie über das Überschreiten der Dispergierfähigkeit der Öle für Feststoffe und Alterungsprodukte hervorgerufen. Hierdurch werden dann die notwendigen Eigenschaften der Öle zum störungsfreien Betrieb der Motoren mitunter drastisch verschlechtert. Eine erhöhte Viskosität hat z.B. beim Startvorgang einen verlängerten Transport des Öles zu den Schmierstellen zur Folge, wodurch ein Anstieg des Verschleißes eintritt.

Der Verbrauch der Dispergieradditive hat eine Verschlechterung der Fähigkeit der Öle zur Reinhaltung der Motoren, insbesondere an kritischen Schmierstellen, wie im Bereich der Kolbenringe/Nuten und Feuerstege, zur Folge, sowie eine Verschlechterung bei der Verhinderung der Ablagerungsbildung an Ventilen und im Ventiltrieb.

Wünschenswert ist es daher, die während des motorischen Betriebes zwangsläufig auftretende Verschlechterung der Eigenschaften der Motorenöle kontinuierlich oder in kurzen Zeitabschnitten, d.h. beispielsweise ein oder mehrmals während eines Betriebes einer Brennkraftmaschine, zu erfassen.

Bis heute ist es jedoch noch nicht gelungen, zuverlässige Meßfühler für eine Ölzustandsanalyse zu entwickeln, wobei für einen längeren Betrieb des Motorenöls im Motor, insbesondere bei instationären Motoren, eine on-board-Analyse, d.h. eine Analyse direkt am Motor, erforderlich ist.

Bisher wurden die unterschiedlichsten Ölsensoren entwickelt, die insbesondere die Viskosität, TAN (Total Acid Number) oder den Füllstand sensieren. Eine besondere Schwierigkeit ist hierbei die Verwendung unterschiedlicher Öle an ein und derselben Brennkraftmaschine sowie das Kompensieren unterschiedlicher Alterungseinflüsse auf die sensierte Eigenschaft. So ist es beispielsweise aus US-A 4,675,662 und 4,721,874, EP 527 176 B und JPN. Appl. Phys., 1993, Acoustic Plate Viskosity Sensor, bekannt, die sich mit der Alterung des Öls geänderte Viskosität als Meßgröße für den Ölzustand heranzuziehen. Dies geschieht über akustische Laufzeitveränderungen, die Phasenverschiebung oder über Eigenfrequenzänderungen eines Schwingquarzes. Problematisch sind hierbei zum einen die häufig fehlende Möglichkeit, die Messung onboard vorzunehmen, und zum anderen die möglichen gegenläufigen Effekte "Abbau des Motorenöls und Verdünnung durch Kraftstoff", die die Viskosität erniedrigen, gegenüber der "Verknüpfung der Abbauprodukte", die die Viskosität erhöhen, solange sie nicht als Schlamm ausfallen.

Die TAN oder auch TBN (Total Basic Number) ist vom Grundprinzip für eine on-board-Messung nicht geeignet, da hierbei das Altöl mit KOH titriert wird. Neuere Ansätze, wie beispielsweise aus SAE 910497, SAE 962112, US-A 4,675,662, 4,792,791 und 5,200,027 bekannt, zeigen hier interessante Lösungen, die beispielsweise mit Kapazitätssensoren, Messung der lonenwanderung oder einer Potentialdifferenz, mit elektrochemischen Festkörperzellen und mit Korrosionssensoren arbeiten. Diese Lösungsansätze sind teils ungenau, noch zu groß und zu schwer oder benötigen ein Opferbauteil, das grundsätzlich unerwünscht ist. Ferner sind noch mathematische Modelle (SAE 870403) und HC-Abgassensoren (DE42 35 225) bekannt, die bislang auch noch nicht zu einem Durchbruch geführt haben. Auch Füllstandssensoren sind wenig geeignet, da diese beispielsweise bei einer starken Verdünnung des Motorenöls durch Kraftstoffe versagen.

In "Molekular Imprinting of Chemically Sensitive Coatings - New Strategies for Sensor Design and Fabrication" von F. L. Dickert, P. Forth, P. Lieberzeit, M. Tortschanoff, W.-E. Bulst, U. Knauer und G. Fischerauer in "Sensor 97" 8. internationale Messe, Nümberg, 1997, wird ein molekulares Prägen für massensensitive Sensorik in Gasen wie in Flüssigkeiten näher beschrieben.

Die US-A 5,151,110 beschreibt Sensoren, die als Molekularsiebe geeignet sind, bestimmte Substanzen in sehr geringen Mengen festzustellen. In dieser Schrift geht es darum, mittels der Sensoren toxische Substanzen wie Umweltgifte und dergleichen zu bestimmen. Beispielhaft werden als Substanzen für diese Sensoren Zeolithe genannt. Die Sensoren sollen für eine Vielzahl unterschiedlichster organischer und anorganischer Verbindungen geeignet sein.

Die DE 43 09 660 A1 beschreibt selektive anorganische Katalysatoren, die zum Beispiel durch ein Sol-Gel-Verfahren erzeugt werden können, wenn das abzudrückende Molekül während der Polymerisation in die sich bildende anorganische Matrix eingeschlossen wird. Die selektiven Katalysatoren können in allen Bereichen der heterogenen Katalyse eingesetzt werden. Sie weisen eine hohe Temperaturstabilität auf und eignen sich zur selektiven Katalyse von inter- und intramolekularen Reaktionen.

Aufgabe der vorliegenden Erfindung ist es, einen Ölqualitätssensor der in Anspruch 8 genannten Gattung zur Verfügung zu stellen, dessen sensitive Schicht aufgrund ihrer chemischen Zusammensetzung für die Bedingungen, bei denen der Ölqualitätssensor eingesetzt wird, insbesondere an einer Brennkraftmaschine, eine besondere Eignung aufweist.

Die Lösung dieser Aufgabe liefert ein erfindungsgemäßer Ölqualitätssensor mit den Merkmalen des Anspruchs 8. Die erfindungsgemäß für die sensitive Schicht verwendete Sol-Gel-Phase, bei der es sich um ein anorganisches Polymer handelt, begünstigt die Resistenz des Sensors unter den in einer Brennkraftmaschine gegebenen Bedingungen, insbesondere der erhöhten Temperatur etc.

Die für die sensitive Schicht des Ölqualitätssensors erfindungsgemäß verwendeten Sol-Gel-Phasen können z.B. durch Hydrolyse von verschiedenen Silanen unter saurer oder alkalischer Katalyse gebildet werden. Als gemeinsames Lösungsmittel für Wasser und die Silane wird insbesondere ein Alkohol verwendet. Bei saurer Katalyse erfolgt nach Hydrolyse eine langsame Polymerisation, die zu linearen Bruchstücken führt.
Durch alkalische Katalyse werden nach relativ schneller Reaktion quervemetzte Polymere erhalten. Die Quervemetzung kann sogar so weit führen, daß es zur Ausbildung von kugelförmigen Produkten kommt. Sensitive Schichten aus Sol-Gel-Phase (anorganische Polymere) begünstigen die Resistenz des Sensors gegenüber schädlichen Umwelteinflüssen wie Hitze oder aggressiven Medien. Dadurch erhöht sich die Standzeit des Sensors, ebenso wie seine Zuverlässigkeit, da die schädlichen Umweltbedingungen im vorgesehenen Einsatzbereich die sensitive Schicht weitgehend nicht schädlich beeinflussen können. Ein weiterer Vorteil der Erfindung ergibt sich durch die Verwendung einer Modellsubstanz als Prägesubstanz für die Sol-Gel-Phase. Als Modellsubstanz ist eine acide Komponente, z.B. eine organische Säure, beispielsweise Caprinsäure besonders gut geeignet.
Die auf diese Weise geprägte sensitive Schicht, lagert mit bemerkenswerter Selektivität entsprechende acide Komponenten, die durch den Gebrauch des Öls entstehen z.B. Carbonsäuren ein, was die Bestimmung der Ölqualität unabhängig von der verwendeten Ölsorte macht, da solche aciden Komponenten durch Oxidation, insbesondere durch thermische Belastung in jedem Öl einer Brennkraftmaschine entstehen.
Besonders geeignet ist eine Sol-Gel-Phase die unter Verwendung einer Komponente hergestellt wird, die eine Aminogruppe enthält. Dies begünstigt die Einlagerung der Prägesubstanz bzw. Modellsubstanz, da polare Wechselwirkungen ausgenutzt werden können.
Durch die Art der Katalyse läßt sich die Struktur der Sol-Gel-Phase weitgehend einstellen. Insbesondere führt eine saure Katalyse nach Hydrolyse zu linearen Bruchstücken, wogegen eine alkalische Katalyse zu quervernetzten Polymeren, bis hin zur Ausbildung von kugelförmigen Produkten führt.
Außerdem bietet sich ein Alkohol als Lösungsmittel an, da er insbesondere für Wasser und Silane als gemeinsames Lösungsmittel fungiert.
Weiterhin liefert die Beschränkung der zur Prägung benutzten Modellsubstanz auf weniger als 25% des Gesamtgewichts der sensitiven Schicht sehr homogene Schichten, da eine Phasentrennung nach dem Verdampfen des Lösungsmittels ausbleibt.

Die Verwendung eines Tetraalkoxysilans, z.B. eines Tetraethoxysilans zur Herstellung der Sol-Gel-Phase erweist sich als besonders vorteilhaft.
Vorzugsweise wird bei der Herstellung weniger als 80% Tetraalkoxysilan verwendet. Dies vermindert die zu starke Vernetzung der Sol-Gel-Phase und beugt so einer Unlöslichkeit und damit Unbrauchbarkeit aufgrund der Ausbildung zu inhomogener Schichten vor. Außerdem erweist sich die Verwendung eines Dialkylaminoalkyltrialkoxysilans bei der Herstellung der Sol-Gel-Phase als besonders vorteilhaft.
Vorteilhafterweise wird bei der Herstellung weniger als 70 Vol% Dialkylaminoalkyltrialkoxysilan verwendet, was sich positiv auf die Festigkeit der sensitiven Schicht auswirkt.

Als Modellsubstanz zum Prägen der sensitiven Schicht eignet sich insbesondere eine aliphatische Carbonsäure, vorzugsweise eine längerkettige aliphatische Carbonsäure, wie beispielsweise Caprinsäure. Es wurde festgestellt, das derartige Modellsubstanzen offenbar eine ähnliche chemische Konstitution aufweisen wie bestimmte bei der thermischen Belastung des Motoröls entstehende acide Komponenten, die zu untersuchen sind. Daher sind solche Verbindungen als Modellsubstanzen besonders geeignet.

Die Herstellung der Sol-Gel-Phase geschieht vorzugsweise ausgehend von wenigstens einer Komponente, die eine Aminogruppe enthält. Dies kann ein aminosubstituiertes Silan sein, beispielsweise ein Dialkylaminoalkyltrialkoxysilan. Zum Beispiel erwies sich Dimethylaminopropyltrimethoxysilan als geeignet.

Weiterhin verwendet man zur Herstellung der Sol-Gel-Phase gemäß einer besonders bevorzugten Variante der Erfindung verschiedene Silane, z.B. ein Tetraalkoxysilan in Verbindung mit einem Dialkylaminoalkyltrialkoxysilan. Je nach Hydrolysebedingungen und je nach Temperatur und Zeitdauer einer anschließenden Aushärtung erhält man Polymere mit unterschiedlichem Vemetzungsgrad. Dabei werden erfindungsgemäß in jedem Fall anorganische, d.h. keramische Silikatpolymere erhalten, da bei der Herstellung die organischen Reste, z.B. Alkohole, abgespalten werden. Diese erfindungsgemäß für die sensitive Schicht des Ölqualitätssensors verwendeten keramischen Polymeren erweisen sich unter den Einsatzbedingungen des Ölqualitätssensors als besonders resistent.

Der erfindungsgemäße Ölqualitätssensor weist gemäß einer Weiterbildung der Erfindung neben der sensitiven Schicht auf einem massensensitiven Bauteil (QMB, SAW, Lamb-Oscillator etc.) eine nicht-sensitive Referenz des jeweils identischen Bauteils auf. Als diese nicht-sensitive Referenz kann ein unbeschichteter Schwingquarz oder ein anderer unbeschichteter massensensitiver Detektor wie z.B. SAW (Surface-acoustic-wave) oder Lamb-Resonator dienen. Alternativ kommt ein entsprechender massensensitiver Detektor in Betracht, der mit einer nicht geprägten Sol-Gel-Phase beschichtet wurde. Der unbeschichtete massensensitive Detektor weist dabei vorzugsweise die gleichen technischen Spezifikationen auf wie der für die Beschichtung mit der sensitiven Schicht vorgesehene massensensitive Detektor. Die nicht sensitive Schicht ist vorzugsweise eine ungeprägte Sol-Gel-Phase mit etwa der gleichen Masse wie die geprägte, als sensitive Schicht verwendete Sol-Gel-Phase. Auf diese Weise wird eine bestmögliche Referenz erhalten.
Gegenstand der Erfindung ist weiterhin ein Verfahren zur Bestimmung der Ölqualität nach dem Oberbegriff des Anspruchs 1. In der DE 197 31 621.2 ist beispielsweise ein solches Verfahren beschrieben. Dort wird unter anderem ausgeführt, daß die Alterung eines Öls unter den Bedingungen einer Brennkraftmaschine mit einer steigenden Acidität einhergeht, d.h., daß eine Zunahme acider Verbindungen festgestellt werden kann. Deshalb wird dort bereits vorgeschlagen, die sensitive Schicht so auszubilden, daß sie sich für den Nachweis der als Abbauprodukte des Öls entstehenden aciden Verbindungen eignet. Dazu werden jedoch in der genannten Schrift organische Polymere, beispielsweise Polyurethane verwendet, die durch den Einbau basischer Komponenten in die Polymerstruktur modifiziert werden. Ein Anliegen der vorliegenden Erfindung ist es, ein Verfahren zur Bestimmung der Ölqualität der genannten Gattung zur Verfügung zu stellen, bei dem die verwendete sensitive Schicht unter den in einer Brennkraftmaschine gegebenen Bedingungen eine besonders günstige Resistenz aufweist.

Die Lösung dieser Aufgabe liefert ein erfindungsgemäßes Verfahren der vorgenannten Gattung mit den kennzeichnenden Merkmalen des Anspruchs 1. Erfindungsgemäß werden als Abbauprodukte des Öls acide Komponenten untersucht, wobei jedoch die sensitive Schicht wenigstens ein keramisches Material umfaßt, das ausgehend von einer Sol-Gel-Phase herstellbar ist, die mit der Modellsubstanz, die eine acide Komponente ist, geprägt wurde.

Die nach dem erfindungsgemäßen Verfahren untersuchte acide Komponente ist vorzugsweise wenigstens eine organische Säure, weiter vorzugsweise wenigstens eine Carbonsäure.

Die Ein- bzw. Auslagerung des Analyten in bzw. aus der sensitiven Schicht wird vorzugsweise bestimmt über die Massenänderung der sensitiven Schicht, insbesondere indem man diese auf einen Schwingquarz aufbringt und die Massenänderung z.B. durch die Änderung der Resonanzfrequenz des beschichteten Schwingquarzes bestimmt. Anstelle eines Schwingquarzes ist erfindungsgemäß ebenfalls bevorzugt die Verwendung eines anderen massensensitiven Detektors wie z.B. SAW (Surface-acoustic-wave) oder Lamb-Resonators.

Das erneute Ein- bzw. Auslagern der zu detektierten Substanzen in die sensitive Schicht läßt sich grundsätzlich mittels z.B. FT-IR anhand der Intensität der spezifischen Absorption im Bereich der asymmetrischen CH₂-Streckschwingung nachweisen.

Bevorzugte Sol-Gel-Phasen für die Herstellung der im Rahmen des erfindungsgemäßen Verfahrens zur Bestimmung der Ölqualität verwendeten sensitiven Schicht sind in den Ansprüchen 9 bis 25 beschrieben.

Nachfolgend wird die Erfindung anhand von Ausführungsbeispielen unter Bezugnahme auf die beiliegenden Diagramme näher beschrieben.

Es zeigen:
- Fig. 1: IR-Spektren einer Sol-Gel Schicht vor und nach dem Auswaschen der Caprinsäure mit Ethanol
- Fig. 2: IR-Spektren einer Sol-Gel Schicht vor und nach der Einlagerung von Neuöl
- Fig. 3: IR-Spektren einer Sol-Gel Schicht vor und nach der Einlagerung von Altöl
- Fig. 4: Frequenzantwort eines beschichteten und unbeschichteten Schwingquarzes beim Wechsel von Neu- auf Altöl

### Beispiel für die Herstellung eines erfindungsgemäßen Ölqualitätssensors

50µl Dimethylaminopropyltrimethoxysilan und 50µl Tetraethoxysilan wurden mit 100 µl Wasser versetzt und in 1800 µl Ethanol gelöst. Als Prägemolekül wurden 20 mg Caprinsäure zugefügt. Die Säure dient außerdem auch als Katalysator und Weichmacher. Die Mischung wurde dann über Nacht stehen gelassen. Danach wurde ein entsprechendes Volumen entweder auf ein Glasplättchen (für die FT-IR-Untersuchungen) oder einen Schwingquarz aufgetragen. Die Mischung ließ man dann bei Raumtemperatur ausreagieren und anschließend bei 100°C im Trockenschrank eine Stunde altern. Für die IR-Untersuchungen wurden die mit ∼2µm beschichteten Glasplättchen zum Auswaschen der Caprinsäure 30 min in Ethanol gelegt und anschließend noch einmal mit 1 ml Ethanol gespült. Die Einlagerung von Neu- bzw. Altöl erfolgte durch legen der Plättchen 30 min lang in das entsprechende Öl. Oberflächlich anhaftendes Öl, das nicht von der Schicht aufgenommen wurde, wurde durch Abspülen mit 2x1 ml n-Heptan pro Plättchenseite entfernt. Der schlechten Löslichkeit des Altöls wurde damit Rechnung getragen, daß zusätzlich jede Seite noch mal mit 1 mt n-Heptan gespült wurde.

### FT-IR Messungen

- FT-IR-Messungen:: Anzahl der Scans: 10
Auflösung: 4 cm⁻¹
Spiegelgeschwindigkeit: 0,4 cm/s
- Netzwerkanalysator-Messungen:: Die Frequenzänderung wurde am Maximum verfolgt.
Anzahl der Punkte: 1601
Spanne: 20 kHz
Smoothing aperture: 1% der Spanne
Bandwidth: 300 Hz
Averaging: 10

Zuerst wurde die Ausbildung von Schichten bei unterschiedlichen Verhältnissen an den beiden Silanen untersucht.
Bei hohen Anteilen an Tetraethoxysilan (80-100 Vol%) kam es vor, daß das entstehende Polymer zu stark vernetzt war und damit unlöslich wurde und ausfiel. Somit konnten nur sehr inhomogene Schichten erhalten werden.
Auf der anderen Seite wieder wurden bei sehr hohen Anteilen an Dimethylaminopropyltrimethoxysilan (70-100 Vol%) die Polymere nicht fest. Auch hier konnten keine Beschichtungen durchgeführt werden. Um einen gewissen Spielraum auf beiden Seiten zu haben wurde deshalb für weitere Untersuchungen immer eine 1:1 v/v Mischung verwendet.
Bei Variation des Gehaltes an Caprinsäure wurde festgestellt, daß der maximale Gehalt vorzugsweise bei ca. 25 Gew% liegt. Oberhalb dieser Menge tritt beim Verdampfen des Lösungsmittels Phasentrennung auf und es bilden sich sehr inhomogene Schichten aus.
Zuerst wurden nun Glasplättchen mit ∼2µm dicken Schichten beschichtet und mit FTIR vermessen. Es wurde nur der Bereich von 3200 bis 2600 cm⁻¹ untersucht, da in diesem Bereich die unterschiedlichen CH- Schwingungen auftreten. Hauptsächlich interessierten hier die asymmetrischen CH₂- Schwingungen, da diese sowohl bei den Ölen als auch bei der Caprinsäure vorhanden sind und an deren Intensität man den unterschiedlichen Grad der Einlagerung gut erkennen kann.

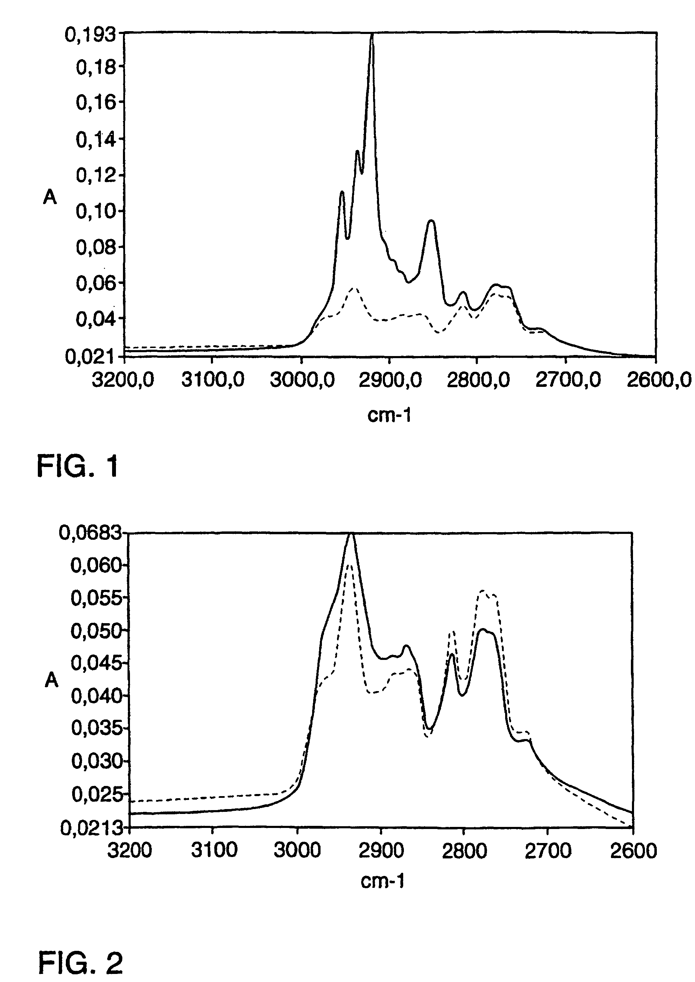

Fig. 1 zeigt die IR-Spektren der Schicht mit Caprinsäure, vor und nach dem Auswaschen mit Ethanol. Man erkennt das Verschwinden der Banden der unterschiedlichen CH-Schwingungen. Die Banden verschwinden nicht vollständig, da ja auch Dimethylaminopropyltrimethoxysilan, das ein Bestandteil der Schicht ist, CH- Bindungen aufweist.

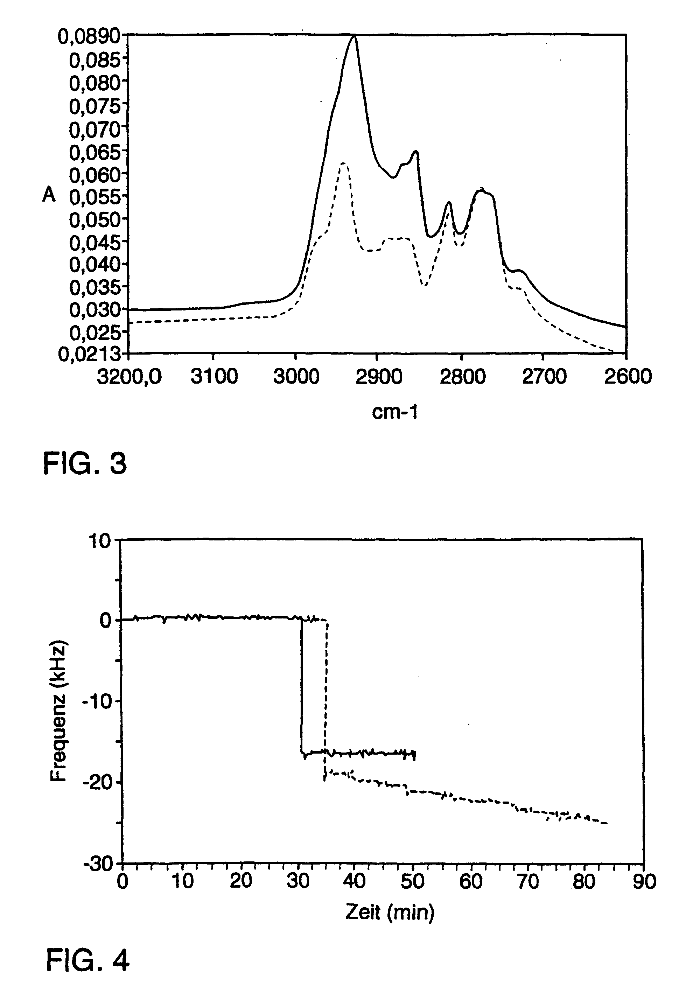

Die folgenden Figuren 2 und 3 zeigen die unterschiedlichen Intensitäten der asymmetrischen CH₂- Schwingungen vor und nach der Einlagerung von Altöl (Fig. 3) und Neuöl (Fig. 2). Hier lagert sich Altöl deutlich besser ein als Neuöl. Dies zeigt deutlich den Erfolg der oben angeführten Strategie.

### Massensensitive Untersuchungen

Ausgestattet mit diesen Ergebnissen wurden nun Messungen mit massensensitiven Bauteilen durchgeführt. Dazu wurde ein unbeschichteter 10 MHz Schwingquarz einmal in Neuöl und dann in Altöl eingetaucht und die damit verbundene Frequenzänderung mit einem Netzwerkanalysator (HP 8752C) aufgenommen. Anschließend wurde auf einem 10 MHz Schwingquarz die Sol-Gel Schicht aufgebracht. Dabei wurde in der Mitte beider Elektroden je 4µl aufgebracht. Die Frequenz änderte sich hierbei von 10,000455 auf 9,983051 MHz. Dies entspricht ungefähr der Massenbeladung einer Schichtdicke von 350 nm pro Seite.
Anschließend wurde die Caprinsäure wie oben angeführt durch Eintauchen und Abspülen mit Ethanol ausgewaschen. Der Schwingquarz hatte nun eine Frequenz von 9,989635 MHz. Die Differenz ist größer als daß sie nur durch auswaschen der Caprinsäure zustande kommen könnte. Möglicherweise wurden hier auch noch unreagierte Monomere ausgewaschen.
Der nun beschichtete QMB wurde wie der unbeschichtete QMB abwechselnd in Neuund Altöl getaucht und die Frequenzänderung mit dem Netzwerkanalysator beobachtet.

Fig. 4 zeigt deutlich, daß der beschichtete Schwingquarz eine deutlich höhere Frequenzänderung zeigt als der Referenzquarz. Die Differenz zwischen den beiden QMB's beträgt -2,5 kHz. Mit höheren Schichtdicken können sicherlich auch noch höhere Effekte erzielt werden. Auch diese Ergebnisse zeigen den Erfolg der angewandten Strategie. Beobachtet man den Verlauf der Frequenzänderung bei Eintauchen des beschichteten QMB in Altöl weiter, sieht man, daß die Einlagerung noch nicht vollständig abgeschlossen ist. Durch das Auffüllen der freien Plätze in tieferen Schichten, erfolgt eine weitere Frequenzemiedrigung, was sich in einer langsamen Signaländerung äußert. Dadurch erhält man eine weitere Verbesserung, die Erhöhung des Effekts.

## Patentansprüche

1. Verfahren zur Bestimmung der Ölqualität, unter Zuhilfenahme einer sensitiven Schicht, die eine an mindestens einen Analyten adaptierte Oberfläche und/oder ein an mindestens einen Analyten adaptiertes Schichtvolumen hat, die bzw. das entsprechend einer vorliegenden Affinität des Analyten zu der Schicht zur analytspezifischen wiederholten Ein- und Auslagerung des Analyten prädestiniert ist, zur Feststellung des Analyten in einem Öl durch Ermittlung einer Massenänderung der sensitiven Schicht, wobei als Analyt wenigstens eine Komponente untersucht wird, welche durch den Gebrauch des Öls entsteht, **dadurch gekennzeichnet, daß** die sensitive Schicht wenigstens ein keramisches Material umfasst, welches herstellbar ist ausgehend von einer Sol-Gel-Phase, die mit einer Modellsubstanz geprägt worden ist, wobei die Modellsubstanz eine acide Komponente ist.

2. Verfahren zur Bestimmung der Ölqualität nach Anspruch 1, **dadurch gekennzeichnet, daß** als Komponente wenigstens eine organische Säure, vorzugsweise wenigstens eine Carbonsäure untersucht wird.

3. Verfahren zur Bestimmung der Ölqualität nach Anspruch 1 oder 2, **dadurch gekennzeichnet, daß** die Massenänderung der sensitiven Schicht dadurch festgestellt wird, daß man diese auf einen massensensitiven Detektor aufbringt.

4. Verfahren zur Bestimmung der Ölqualität nach Anspruch 3, **dadurch gekennzeichnet, daß** man als massensensitiven Detektor einen QMB (Quartz-Micro-Balance), einen SAW (Surface-acoustic-wave) oder Lamb-Resonator verwendet.

5. Verfahren zur Bestimmung der Ölqualität nach einem der Ansprüche 1 bis 4, **dadurch gekennzeichnet, daß** man die Massenänderung feststellt über die Änderung der Resonanzfrequenz eines mit der sensitiven Schicht beschichteten massensensitiven Detektors.

6. Verfahren zur Bestimmung der Ölqualität nach einem der Ansprüche 1 bis 5, **dadurch gekennzeichnet, daß** zur erweiterten Messung der Ölqualität eine zweite, nicht-sensitive Schicht verwendet wird.

7. Verfahren zur Bestimmung der Ölqualität nach Anspruch 6, **dadurch gekennzeichnet, daß** die Differenz der Frequenzantwort zwischen sensitiver und nichtsensitiver Schicht bzw. unbeschichtetem massensensitiven Detektor gemessen wird.

8. Ölqualitätssensor, insbesondere für eine Brennkraftmaschine, mit einer sensitiven Schicht, die eine speziell an mindestens einen in einem Öl vorliegenden Bestandteil adaptierte Oberfläche oder ein Schichtvolumen hat, die bzw. das entsprechend einer vorliegenden Affinität des Bestandteils zu der Schicht zur spezifischen Wiedereinlagerung des Bestandteils prädestiniert ist, **dadurch gekennzeichnet, daß** die sensitive Schicht herstellbar ist ausgehend von einer Sol-Gel-Phase, die mit einer Modellsubstanz geprägt worden ist, wobei die Modellsubstanz eine acide Komponente ist, wobei die sensitive Schicht eine keramische Schicht ist.

9. Ölqualitätssensor nach Anspruch 8, **dadurch gekennzeichnet, daß** es sich bei der Modellsubstanz um eine organische Säure handelt.

10. Ölqualitätssensor nach einem der Ansprüche 8 oder 9, **dadurch gekennzeichnet, daß** es sich bei der Modellsubstanz um eine Carbonsäure handelt.

11. Ölqualitätssensor nach einem der Ansprüche 8 bis 10, **dadurch gekennzeichnet, daß** es sich bei der Modellsubstanz um eine aliphatische Carbonsäure handelt.

12. Ölqualitätssensor nach einem der Ansprüche 8 bis 11, **dadurch gekennzeichnet, daß** es sich bei der Modellsubstanz um eine längerkettige aliphatische Carbonsäure, vorzugsweise Caprinsäure handelt.

13. Ölqualitätssensor nach einem der Ansprüche 8 bis 12, **dadurch gekennzeichnet, daß** die Sol-Gel-Phase herstellbar ist, ausgehend von wenigstens einer Komponente die eine Aminogruppe enthält.

14. Ölqualitätssensor nach einem der Ansprüche 8 bis 13, **dadurch gekennzeichnet, daß** die Sol-Gel-Phase durch Hydrolyse von Silanen unter saurer oder alkalischer Katalyse herstellbar ist.

15. Ölqualitätssensor nach einem der Ansprüche 8 bis 14, **dadurch gekennzeichnet, daß** die Sol-Gel-Phase durch Anwendung einer höheren Temperatur zur Aushärtung unter Bildung quervernetzter Polymere herstellbar ist.

16. Ölqualitätssensor nach einem der Ansprüche 8 bis 15, **dadurch gekennzeichnet, daß** die Sol-Gel-Phase unter Anwendung eines Alkohols als gemeinsames Lösungsmittel für Wasser und Silane herstellbar ist.

17. Ölqualitätssensor nach einem der Ansprüche 8 bis 16, **dadurch gekennzeichnet, daß** die Sol-Gel-Phase in ihrem unausgewaschenen Zustand weniger als 25 Gew% der Modellsubstanz enthält.

18. Ölqualitätssensor nach einem der Ansprüche 8 bis 17, **dadurch gekennzeichnet, daß** die Sol-Gel-Phase herstellbar ist ausgehend von wenigstens einem Tetraalkoxysilan, vorzugsweise Tetraethoxysilan.

19. Ölqualitätssensor nach einem der Ansprüche 8 bis 18, **dadurch gekennzeichnet, daß** der Anteil an Tetraalkoxysilan, vorzugsweise Tetraethoxysilan bei der Herstellung der Sol-Gel-Phase geringer als 80 Vol% ist.

20. Ölqualitätssensor nach einem der Ansprüche 8 bis 19, **dadurch gekennzeichnet, daß** die Sol-Gel-Phase herstellbar ist, ausgehend von wenigstens einem Dialkylaminoalkyltrialkoxysilan, vorzugsweise Dimethylaminopropyltrimethoxysilan.

21. Ölqualitätssensor nach Anspruch 20, **dadurch gekennzeichnet, daß** der Anteil an Dialkylaminoalkyltrialkoxysilan bei der Herstellung der Sol-Gel-Phase geringer als 70 Vol% ist.

22. Ölqualitätssensor nach einem der Ansprüche 8 bis 21, **dadurch gekennzeichnet, daß** der Ölqualitätssensor neben der sensitiven Schicht eine nicht-sensitive Referenz aufweist.

23. Ölqualitätssensor nach Anspruch 22, **dadurch gekennzeichnet, daß** es sich bei der nichtsensitiven Referenz um einen unbeschichteten massensensitiven Detektor vorzugsweise Schwingquarz oder einen mit einer nicht geprägten Sol-Gel-Phase beschichteten Schwingquarz handelt.

24. Ölqualitätssensor nach Anspruch 23, **dadurch gekennzeichnet, daß** der unbeschichtete Detektor die gleichen technischen Spezifikationen wie der für die Beschichtung mit der sensitiven Schicht vorgesehene Detektor aufweist.

25. Ölqualitätssensor nach einem der Ansprüche 22 bis 24, **dadurch gekennzeichnet, daß** die nicht sensitive Schicht eine ungeprägte Sol-Gel-Phase mit etwa der gleichen Masse wie die geprägte, als sensitive Schicht verwendete Sol-Gel-Phase aufweist.

## Claims

1. Process for determining the quality of oil, with the aid of a sensitive layer which has a surface adapted to at least one analyte and/or a layer volume adapted to at least one analyte, each of which is preorganized for analyte-specific repeated incorporation and release of the analyte in accordance with an existing affinity of the analyte for the layer, for determining the analyte in an oil by determining a change in mass of the sensitive layer, the analyte investigated being at least one component which is formed by the use of the oil, **characterized in that** the sensitive layer comprises at least one ceramic material which can be produced starting from a sol-gel phase which has been imprinted with a model substance, the model substance being an acidic component.

2. Process for determining the quality of oil according to Claim 1, **characterized in that** the component investigated is at least one organic acid, preferably at least one carboxylic acid.

3. Process for determining the quality of oil according to Claim 1 or 2, **characterized in that** the change in mass of the sensitive layer is detected by applying it to a mass-sensitive detector.

4. Process for determining the quality of oil according to Claim 3, **characterized in that** the mass-sensitive detector used is a QMB (quartz microbalance), an SAW (surface acoustic wave) or Lamb resonator.

5. Process for determining the quality of oil according to one of Claims 1 to 4, **characterized in that** the change in mass is detected via the change in the resonance frequency of a mass-sensitive detector coated with the sensitive layer.

6. Process for determining the quality of oil according to one of Claims 1 to 5, **characterized in that** the analysis of the quality of oil is extended by using a second, insensitive layer.

7. Process for determining the quality of oil according to Claim 6, **characterized in that** the difference in the frequency response between sensitive and insensitive layer or uncoated mass-sensitive detector is measured.

8. Oil quality sensor, especially for an internal combustion engine, having a sensitive layer which has a surface which is adapted specifically to at least one constituent present in an oil, or a layer volume, each of which is preorganized for specific reincorporation of the constituent in accordance with an existing affinity of the constituent for the layer, **characterized in that** the sensitive layer can be produced starting from a sol-gel phase which has been imprinted with a model substance, the model substance being an acidic component and the sensitive layer being a ceramic layer.

9. Oil quality sensor according to Claim 8, **characterized in that** the model substance is an organic acid.

10. Oil quality sensor according to one of Claims 8 or 9, **characterized in that** the model substance is a carboxylic acid.

11. Oil quality sensor according to one of Claims 8 to 10, **characterized in that** the model substance is an aliphatic carboxylic acid.

12. Oil quality sensor according to one of Claims 8 to 11, **characterized in that** the model substance is a relatively long-chain aliphatic carboxylic acid, preferably capric acid.

13. Oil quality sensor according to one of Claims 8 to 12, **characterized in that** the sol-gel phase can be prepared starting from at least one component which contains an amino group.

14. Oil quality sensor according to one of Claims 8 to 13, **characterized in that** the sol-gel phase can be prepared by hydrolysing silanes under acidic or alkaline catalysis.

15. Oil quality sensor according to one of Claims 8 to 14, **characterized in that** the sol-gel phase can be prepared by using a higher temperature for curing to form crosslinked polymers.

16. Oil quality sensor according to one of Claims 8 to 15, **characterized in that** the sol-gel phase can be prepared using an alcohol as a common solvent for water and silanes.

17. Oil quality sensor according to one of Claims 8 to 16, **characterized in that** the sol-gel phase in its unwashed state contains less than 25% by weight of the model substance.

18. Oil quality sensor according to one of Claims 8 to 17, **characterized in that** the sol-gel phase can be prepared starting from at least one tetraalkoxysilane, preferably tetraethoxysilane.

19. Oil quality sensor according to one of Claims 8 to 18, **characterized in that** the proportion of tetraalkoxysilane, preferably tetraethoxysilane, in the preparation of the sol-gel phase is less than 80% by volume.

20. Oil quality sensor according to one of Claims 8 to 19, **characterized in that** the sol-gel phase can be prepared starting from at least one dialkylaminoalkyltrialkoxysilane, preferably dimethylaminopropyltrimethoxysilane.

21. Oil quality sensor according to Claim 20, **characterized in that** the proportion of dialkylaminoalkyltrialkoxysilane in the preparation of the sol-gel phase is less than 70% by volume.

22. Oil quality sensor according to one of Claims 8 to 21, **characterized in that** the oil quality sensor has an insensitive reference in addition to the sensitive layer.

23. Oil quality sensor according to Claim 22, **characterized in that** the insensitive reference is an uncoated mass-sensitive detector, preferably crystal oscillator or a crystal oscillator coated with an unimprinted sol-gel phase.

24. Oil quality sensor according to Claim 23, **characterized in that** the uncoated detector has the same technical specifications as the detector provided for the coating with the sensitive layer.

25. Oil quality sensor according to one of Claims 22 to 24, **characterized in that** the insensitive layer has an unimprinted sol-gel phase having about the same mass as the imprinted sol-gel phase used as the sensitive layer.

## Revendications

1. Procédé de détermination de la qualité d'une huile par recours à une couche sensible qui présente une surface adaptée à au moins un analyte et/ou un volume de couche adapté à au moins un analyte qui est prédestiné(e) à incorporer et rejeter de manière répétée et spécifique à l'analyte en fonction de l'affinité que l'analyte présente vis-à-vis de la couche, pour déterminer l'analyte dans une huile par détermination d'une modification de la masse de la couche sensible, au moins un composant qui apparaît par usure de l'huile étant analysé en tant qu'analyte, **caractérisé en ce que** la couche sensible comprend au moins un matériau céramique qui peut être fabriqué à partir d'une phase de sol-gel dans laquelle est imprimée une substance de modèle, la substance de modèle étant un composant acide.

2. Procédé de détermination de la qualité d'une huile selon la revendication 1, **caractérisé en ce que** comme composant, on analysé au moins un acide organique et de préférence au moins un acide carboxylique.

3. Procédé de détermination de la qualité d'une huile selon la revendication 1 ou 2, **caractérisé en ce que** la modification de la masse de la couche sensible est déterminée en appliquant cette dernière sur un détecteur sensible à la masse.

4. Procédé de détermination de la qualité d'une huile selon la revendication 3, **caractérisé en ce que** comme détecteur sensible à la masse on utilise une QMB ("Quartz-Micro-Balance" - microbalance à quartz), une SAW ("Surface-acoustic-wave" - onde acoustique de surface) ou un résonateur Lamb.

5. Procédé de détermination de la qualité d'une huile selon l'une des revendications 1 à 4, **caractérisé en ce que** l'on détermine la modification de masse par l'intermédiaire de la modification de la fréquence de résonance d'un détecteur sensible à la masse revêtu de la couche sensible.

6. Procédé de détermination de la qualité d'une huile selon l'une des revendications 1 à 5, **caractérisé en ce que** pour mesurer davantage la qualité de l'huile, on utilise une deuxième couche, non sensible.

7. Procédé de détermination de la qualité d'une huile selon la revendication 6, **caractérisé en ce que** l'on mesure la différence des réponses en fréquence de la couche sensible et de la couche non sensible ou du détecteur sensible à la masse, non revêtu.

8. Détecteur de qualité d'huile, notamment pour un moteur à combustion interne, avec une couche sensible qui présente une surface ou un volume de couche spécialement adapté(e) à au moins un constituant présent dans l'huile qui est prédestiné(e) à incorporer de nouveau de manière spécifique au constituant en fonction de l'affinité que le constituant présente vis-à-vis de la couche, **caractérisé en ce que** la couche sensible peut être préparée à partir d'une phase de sol-gel dans laquelle est imprimée une substance de modèle, la substance de modèle étant un composant acide et la couche sensible étant une couche céramique.

9. Détecteur de qualité d'huile selon la revendication 8, **caractérisé en ce que** la substance de modèle est un acide organique.

10. Détecteur de qualité d'huile selon l'une des revendications 8 ou 9, **caractérisé en ce que** la substance de modèle est un acide carboxylique.

11. Détecteur de qualité d'huile selon l'une des revendications 8 à 10, **caractérisé en ce que** la substance de modèle est un acide carboxylique aliphatique.

12. Détecteur de qualité d'huile selon l'une des revendications 8 à 11, **caractérisé en ce que** la substance de modèle est un acide carboxylique aliphatique à longue chaîne, de préférence l'acide caprique.

13. Détecteur de qualité d'huile selon l'une des revendications 8 à 12, **caractérisé en ce que** la phase sol-gel peut être préparée à partir d'au moins un composant qui contient un groupe amino.

14. Détecteur de qualité d'huile selon l'une des revendications 8 à 13, **caractérisé en ce que** la phase sol-gel peut être préparée par hydrolyse de silanes sous catalyse acide ou alcaline.

15. Détecteur de qualité d'huile selon l'une des revendications 8 à 14, **caractérisé en ce que** la phase sol-gel peut être préparée par application d'une haute température de durcissement avec formation de polymères réticulés transversalement.

16. Détecteur de qualité d'huile selon l'une des revendications 8 à 15, **caractérisé en ce que** la phase sol-gel peut être préparée en utilisant un alcool comme solvant commun de l'eau et des silanes.

17. Détecteur de qualité d'huile selon l'une des revendications 8 à 16, **caractérisé en ce que** dans son état non lessivé, la phase sol-gel contient moins de 25 % en poids de la substance de modèle.

18. Détecteur de qualité d'huile selon l'une des revendications 8 à 17, **caractérisé en ce que** la phase sol-gel peut être préparée à partir d'au moins un tétraalcoxysilane, de préférence le tétraéthoxysilane.

19. Détecteur de qualité d'huile selon l'une des revendications 8 à 18, **caractérisé en ce que** lors de la préparation de la phase sol-gel, la proportion de tétraàlcoxysilane, de préférence le tétraéthoxysilane, est inférieure à 80 % en volume.

20. Détecteur de qualité d'huile selon l'une des revendications 8 à 19, **caractérisé en ce que** la phase sol-gel peut être préparée à partir d'au moins un dialkylaminoalkyltrialcoxysilane, de préférence le diméthylaminopropyltriméthoxysilane.

21. Détecteur de qualité d'huile selon la revendication 20, **caractérisé en ce que** lors de la préparation de la phase sol-gel, la proportion de dialkylaminoalkyltrialcoxysilane est inférieure à 70 % en volume.

22. Détecteur de qualité d'huile selon l'une des revendications 8 à 21, **caractérisé en ce qu'**en plus de la couche sensible, le détecteur de qualité d'huile présente une référence non sensible.

23. Détecteur de qualité d'huile selon la revendication 22, **caractérisé en ce que** la référence non sensible est un détecteur sensible à la masse, non revêtu, de préférence un quartz oscillant ou un quartz oscillant revêtu d'une phase sol-gel non imprimée.

24. Détecteur de qualité d'huile selon la revendication 23, **caractérisé en ce que** le détecteur non revêtu présente les mêmes spécifications techniques que le détecteur destiné à être revêtu de la couche sensible.

25. Détecteur de qualité d'huile selon l'une des revendications 22 à 24, **caractérisé en ce que** la couche non sensible est une phase sol-gel non imprimée dont la masse correspond sensiblement à celle de la phase sol-gel imprimée qui est utilisée comme couche sensible.
